# EUROPÄISCHE PATENTANMELDUNG

(11) **EP 2 425 869 A1**
(43) Veröffentlichungstag der Anmeldung: **07.03.2012**
(21) Anmeldenummer: 10175668.2
(22) Anmeldetag: 07.09.2010
(51) Int. Cl.: A61M 16/01, A61M 16/12

(54) **Beatmungsgerät und/oder Anästhesiegerät**

(71) Anmelder: IMT AG, 9470 Buchs (CH)
(72) Erfinder: Friberg, Harri, 9493 Mauren (LI); Däscher, Jakob, 7306 Fläsch (CH)
(74) Vertreter: Patentbüro Paul Rosenich AG

(57) **Zusammenfassung**

Die Erfindung betrifft ein Beatmungsgerät (14) und/oder Anästhesiegerät mit einer Mischvorrichtung zum Mischen eines unter Druck stehenden medizinischen Gases, z.B. Sauerstoff, mit durch ein Gebläse (1) angesaugter Luft, umfassend eine Gasleitung (10) für das medizinische Gas, und eine Luftleitung (11), die in einer gemeinsamen Atemgasleitung (12) münden, dadurch gekennzeichnet, dass sich die Einmündung der Gasleitung (10) in die gemeinsame Atemgasleitung (12) stromabwärts des Gebläses (1) befindet und dass die Gasleitung (10) ein Regelventil (8), vorzugsweise ein Proportionalventil, zur variablen Einstellung des Gasflusses aufweist und dass in der gemeinsamen Atemgasleitung (12) ein Regelventil (4), vorzugsweise ein Proportionalventil, zur Dosierung des Atemgases vorgesehen ist.

## Beschreibung

Die Erfindung bezieht sich auf ein Beatmungsgerät und/oder Anästhesiegerät mit einer Mischvorrichtung zum Mischen eines unter Druck stehenden medizinischen Gases, z.B. Sauerstoff, mit durch ein Gebläse angesaugter Luft, umfassend eine Gasleitung für das medizinische Gas, und eine Luftleitung, die in einer gemeinsamen Atemgasleitung münden.

Die Erfindung bezieht sich auch auf ein Verfahren zum Betrieb eines Beatmungsgeräts und/oder Anästhesiegeräts, bei dem ein Atemgas durch Mischen eines unter Druck stehenden medizinischen Gases, z.B. Sauerstoff, mit durch ein Gebläse geförderter Luft bereitgestellt wird, umfassend eine Gasleitung für das medizinische Gas, und eine Luftleitung, die in einer gemeinsamen Atemgasleitung münden.

Bekannte Beatmungsgeräte mischen Sauerstoff und Luft, wobei beide Gase unter Hochdruck (ca. 2 bis 7 bar) stehen und entweder über ein Hochdruckleitungssystem des Krankenhauses oder aus Gasflaschen entnommen werden. Die beiden Gase werden in einem bestimmten Verhältnis miteinander gemischt (z.B. 1:1) und dem Patienten während des Atemzyklus zugeführt. Die Mischung erfolgt in der Regel dadurch, dass die Gase in einem der Beatmungsmaske oder dem Beatmungsschlauch vorgeschalteten Mischtank (üblicherweise über on/off Ventile) eingespeist und von dort über ein gesteuertes Beatmungsventil zum Patienten weitergeleitet werden.

Es gibt auch Varianten, bei denen die beiden Gase - aus zwei gesonderten Druckleitungen kommend - erst unmittelbar beim Auslass des Beatmungsgerätes gemischt und über zwei gesteuerte Beatmungsventile (Proportionalventile), jeweils eines für die Druckluft und eines für den Hochdruck-Sauerstoff, dem Patienten zugeführt werden. Beide Proportionalventile dosieren synchron die Gasflüsse zum Patienten, so dass daraus die gewünschte Gasmenge und das gewünschte Gasgemisch resultiert.

Diese Methoden funktionieren jedoch nur dann fehlerfrei, wenn für beide Gase ein Hochdrucksystem von etwa gleichem Druck bereitgestellt werden kann. Ein grundlegender Nachteil liegt darin begründet, dass überhaupt Druckluft erforderlich ist. Darüber hinaus erzeugt die Entspannung der Druckluft Kälte. Daher müssen bei externer Anfeuchtung der Atemluft die Patientenschläuche geheizt werden, da sonst Feuchtigkeit auskondensiert.

Bei einer neuen Art von Beatmungsgeräten wird der Luftstrom bzw. Luftdruck über ein Gebläse in Form eines Lüfterrads (auch Blower oder Turbine genannt) erzeugt. Der Vorteil hierbei ist, das Luft nicht in Form von Pressluft zur Verfügung stehen muss, sondern einfach aus der Umgebung angesaugt werden kann. Um bei solchen Systemen Sauerstoff (oder ein anderes Gas oder Gasgemisch) aus einem Hochdruckreservoir (Krankenhausnetz oder Sauerstoffflasche) beizumischen, wird Sauerstoff über ein Ventil in den Ansaugbereich des Blowers geblasen, so dass der Blower neben Luft auch Sauerstoff zum Beatmungsventil vor der Beatmungsmaske leitet.

Dieser Aufbau ist jedoch problematisch. Zwar löst er das Problem der Zumischung von Hochdruck-Sauerstoff an eine relativ niederdruckige Luftquelle, weist dabei aber folgende Nachteile auf:

Der reine Sauerstoff wird über die relativ heissen Gebläseeinrichtungen geführt, wodurch eine stark erhöhte Brandgefahr entsteht. Der Sauerstoff kann nicht genau dosiert werden, was in mangelhafter Beatmung und/oder Verschwendung von relativ teurem Sauerstoff resultiert. Wenn der Patient nicht einatmet, wird unnötig viel Sauerstoff geliefert und verschwendet.

Im folgenden wird der Stand der Technik anhand einiger Dokumente näher erläutert:

Die US 4 022 234 A offenbart ein Beatmungssystem, bei dem zwei verschiedene Gase in einem bestimmten Verhältnis miteinander vermischt werden, bevor sie in die Atemgasleitung geführt werden. Zu diesem Zweck ist eine Druckkammer vorgesehen, in die nacheinander die verschiedenen Gase eingeführt werden. Sobald der Druck in der Druckkammer auf einen bestimmten Wert sinkt, wird zunächst Gas A eingeführt. Erhöht sich dadurch der Druck auf einen weiteren Schwellwert, wird anschließend Gas B eingeführt, bis ein vorbestimmter Enddruck erreicht ist. Sobald dieser Enddruck erreicht ist, wird die Gasmischung in der Druckkammer ausgelassen und einem Reservoir zugeführt, von das Atemgas entnommen wird.

Eine derartige Gasmischung ist mit Nachteilen behaftet, weil keine kontinuierlich erfolgende Mischung von Gas möglich ist. Außerdem kann die Druckmessung fehlerbehaftet sein, so dass keine exakte Mischung der Gase nach einem gewünschten Konzentrationswert erfolgen kann. Darüber hinaus erfordert das System ein zusätzliches Reservoir zur Druckkammer, wodurch sich der Platzbedarf wesentlich erhöht.

Die US 4 587 967 A offenbart ein Kolben-Beatmungsgerät mit variablem Fluss, bei dem Sauerstoff aus einem Sauerstofftank (Sauerstoffflasche) zugeführt wird. In der Sauerstoffleitung befindet sich ein Regelventil (Proportionalventil), das einen Sauerstofffluss proportional zu einem pulsweitenmodulierten Signal erzeugt. Der Sauerstoff gelangt in eine Mischkammer, in die aus einer anderen Leitung Luft aus einer Kolbenpumpe geleitet wird. Aus der Mischkammer gelangt das Atemgas direkt in den Patienten, wobei zwischen Mischkammer und Patient einen Sensor vorgesehen ist, der den Sauerstoff-Gehalt des Atemgases bestimmt. Alle Sensoren sind mit einer Steuerung verbunden, ebenso die Kolbenpumpe.

Der Nachteil eines derartigen Systems besteht darin, dass die Messung des Sauerstoffgehaltes mit Fehlern behaftet sein kann und stark von Temperatur, Feuchtigkeit, Gasfluss und dergleichen abhängig ist. Auch kann über das Kolbensystem kein kontinuierlicher Gasfluss zur Verfügung gestellt werden. Die mengenmäßige Dosierung des Atemgases ist daher nicht fein und genau justierbar.

Die US 5 383 449 A offenbart ein Steuersystem zum Mischen und intermittierenden Zuführen von Gas. Dieses System verwendet zwei unter Druck stehende Gasquellen, Sauerstoff und Luft. Die Zufuhrleitungen der beiden Gase münden in einem großen Druckbehälter, in der eine Vermischung der Gase erfolgt. Das Atemgas wird diesem Druckbehälter in erforderlicher Menge entnommen.

Nachteilig ist insbesondere der große Druckbehälter zur Speicherung des Gasgemisches, wodurch der Platzbedarf und das Gewicht sehr groß wird.

Der Erfindung liegt die Aufgabe zugrunde, ein neues Beatmungsgerät zu schaffen, das einerseits mit der bekannten Niederdruck-Blowertechnologie andererseits mit bekanntem Hochdruck-Sauerstoff arbeitet, jedoch ohne die erwähnte Brandgefahr, ohne Verschwendung von Sauerstoff und unter genauer Dosierung der Mischung zwischen Luft und Sauerstoff oder einem anderen medizinischen Gas, wie z.B. Narkotika, Stickstoff, CO₂ und dgl. Die Erfindung setzt sich insbesondere zum Ziel, die im Stand der Technik auftretenden Nachteile zu beseitigen und ein Beatmungsgerät und/oder Anästhesiegerät bereitzustellen, mit dem die Beimengung eines medizinischen Gases zu Luft exakt gesteuert werden kann. Darüber hinaus soll auch die Mengendosierung des Atemgases zum Patienten hin optimal regelbar sein. Das neue System soll insbesondere gewährleisten, dass die Sauerstoffkonzentration im Atemgas unabhängig von Einflüssen des Atemzyklus konstant gehalten wird. Die Erfindung soll weiters bewirken, dass der Platzbedarf und das Gewicht des Beatmungsgeräts gering gehalten werden und die Sicherheit erhöht wird.

Diese Ziele werden mit einem Beatmungsgerät und/oder Anästhesiegerät der eingangs genannten Art dadurch erreicht, dass sich die Einmündung der Gasleitung in die gemeinsame Atemgasleitung stromabwärts des Gebläses befindet und dass die Gasleitung ein Regelventil, vorzugsweise ein Proportionalventil, zur variablen Einstellung des Gasflusses aufweist und dass in der gemeinsamen Atemgasleitung ein Regelventil, vorzugsweise ein Proportionalventil, zur Dosierung des Atemgases vorgesehen ist.

Diese Maßnahme ermöglicht eine exakt regelbare Zufuhr eines medizinischen Gases zur Luft bei gleichzeitiger genauer Mengendosierung des Atemgases. Dabei erfolgt eine Mischung eines unter Hochdruck stehenden medizinischen Gases mit Luft niedrigen Drucks, die aus der Umgebung angesaugt wird. Auch wenn im folgenden manchmal der Begriff "Sauerstoff" verwendet wird, so ist die Erfindung auf jedes medizinische Gas anwendbar. Bei der Erfindung strömt nur Luft durch das Gebläse, wodurch kein medizinisches Gas in Richtung Lufteinlass verloren gehen kann. Die gewünschte Gaszusammensetzung kann so noch genauer erfolgen.

Bei entsprechender Steuerung lässt sich die Sauerstoffzufuhr in Abhängigkeit von Messparametern eindeutig regeln. Die Messparameter beziehen sich dabei entweder auf systeminterne Parameter, wie Durchflussmengen in den Leitungen oder patientenspezifische Parameter, wie den Atemzyklus. Dadurch kann Sauerstoff nicht nur exakt beigemengt, sondern auch effizient gespart werden, wenn er nicht benötigt wird. In der Ausatemphase kann dieses Regelventil in der Gasleitung gedrosselt werden. Ein inspirationsseitiges Regel- bzw. Proportionalventil sorgt für die optimale Mengendosierung des Gasgemisches in Richtung Patient. Das Zusammenwirken der beiden Regelventile liefert ein präzise abgestimmtes Gasgemisch. Die Erfindung zeichnet sich durch kleinste Bauweise aus, da kein Tank nötig ist. Es ergibt sich ein hoher Spitzenfluss für den Patienten, da das Gebläse (Blower) viel Fluss liefert, egal nach welcher Soll-Sauerstoff-Konzentration geregelt wird.

Es kann sich sowohl um Heimbeatmungsgeräte, als auch um auf Intensivstationen und in Operationssälen verwendete Beatmungsgerät und/oder Anästhesiegeräte handeln. Weitere Druck- und/oder Gasfluss- und/oder Volumensensoren können den Fluss zum Patienten beeinflussen.

In einer Ausführung ist in der Gasleitung ein Durchflusssensor zur Messung des Gasflusses vorgesehen und in der Atemgasleitung ein Durchflusssensor zur Messung des Atemgasflusses vorgesehen. Die Messwerte der einzelnen Durchflusssensoren können zueinander in Beziehung gebracht werden, sodass z.B. aus dem Verhältnis der jeweiligen Durchflussmengen genaue Rückschlüsse auf die Konzentration des medizinischen Gases in der Luft gezogen werden können. In Abhängigkeit dieser Messwertverarbeitung kann nun das Regelventil in der Gasleitung gesteuert werden. Über eine Rückkopplung (Feedbackschleife) kann eine optimale Ventilstellung angefahren werden, die einer bestimmten Gaskonzentration entspricht.

In einer Ausführung ist in der Gasleitung eingangsseitig ein Ventil zur Reduktion des Gasdruckes vorgesehen, wobei das Regelventil stromabwärts davon angeordnet ist. Diese Maßnahme bringt den Vorteil mit sich, dass der Druck des medizinischen Gases vor dem Regelventil im Vergleich zum Hochdruckreservoir bereits stark reduziert ist, wodurch die Regelung des Gasdurchflusses vereinfacht und genauer wird.

In einer Ausführung ist das Ventil zur Reduktion des Gasdruckes variabel einstellbar. Dies ermöglicht eine kontinuierliche Ansteuerung des Ventils und eine beliebige Einstellung des Druckes in der Gasleitung vor dem Regelventil. Das Drosseln dieses Reduktionsventils ist in folgenden Phasen vorteilhaft: Wenn kein Atemzug am Beatmungsventil, z.B. bei Atempausen, anliegt und das Regelventil gedrosselt bzw. vollständig geschlossen wird, würde sich vor dem gedrosselten Regelventil ein höherer Druck aufbauen. Bei erneutem Öffnen des Regelventils sorgt der Druckausgleich dafür, dass übermäßig viel Sauerstoff in die Atemgasleitung strömt, wodurch eine überhöhte Sauerstoffkonzentration im Atemgas entstünde. Wird jedoch das Reduktionsventil zeitgleich mit dem Regelventil gedrosselt, kann ein Überdruck und ein Sauerstoffüberschuss verhindert werden. Dies kann ebenso wie die Auswertung entsprechender Messparameter in Echtzeit erfolgen.

In einer Ausführung ist in der Gasleitung, vorzugsweise zwischen dem Ventil zur Reduktion des Gasdruckes und dem Regelventil zur variablen Einstellung des Gasflusses, ein Sicherheitsventil vorgesehen, wodurch die Sicherheit für Patient und Personal weiter erhöht werden kann.

In einer Ausführung ist das Gebläse in der Luftleitung angeordnet, wodurch das medizinische Gas mit dem durch den Betrieb erwärmten Gebläseteilen nicht in Kontakt kommen kann. Dies ist insbesondere bei Sauerstoff relevant, da dadurch die Brandgefahr wesentlich reduziert werden kann.

In einer Ausführung ist in der Luftleitung zwischen dem Gebläse und der Einmündung der Luftleitung in die Atemgasleitung ein Rückschlagventil vorgesehen, das den Rückstrom von Gas in Richtung Gebläse verhindert. Dies vermindert die Brandgefahr bei Sauerstoff noch mehr, da auch bei veränderten Druckverhältnissen ein Rückstrom von Sauerstoff in Richtung Gebläse unterbunden werden kann. Außerdem wird dadurch verhindert, dass Sauerstoff nach außen gelangt und verschwendet wird.

In einer Ausführung ist in der Atemgasleitung ausgangsseitig ein Drucksensor vorgesehen. Mit diesem Drucksensor lässt sich der Druck der Atemgases kontrollieren, bevor es dem Patienten zur Verfügung gestellt wird. Außerdem kann dieser Drucksensor zur Detektion des Atemzyklus herangezogen werden, der wiederum als Steuergröße für die Regelventile dient.

In einer Ausführung wird die Förderung des medizinischen Gases in die Atemgasleitung ausschließlich durch den, gegebenenfalls reduzierten, Eigendruck des in die Gasleitung eintretenden Gases bewirkt wird. Es sind somit in der Gasleitung keine aktiven Fördereinrichtungen für das medizinische Gas vonnöten. Das unter Hochdruck stehende Gas, das an das Beatmungsgerät angeschlossen ist, nutzt den Eigendruck aus, um in Richtung Atemgasleitung gefördert zu werden.

In einer Ausführung sind das Regelventil und die Durchflussmesser des Beatmungsgeräts und/oder Anästhesiegeräts mit einer Steuereinheit verbunden, die das Regelventil in Abhängigkeit der Messwerte des Durchflusssensors in der Gasleitung und des Durchflusssensors in der Atemgasleitung steuert. Die Durchflussmengen bzw. das Verhältnis der Durchflussmengen zueinander wird als Steuergröße für das Regelventil in der Gasleitung verwendet. Die Messdaten werden ständig in Echtzeit ausgewertet, gegebenenfalls mit Kalibriertabellen abgeglichen und zueinander in Beziehung gesetzt und bilden danach den Ausgangspunkt für eine Echtzeit-Ansteuerung des Regelventils. Bei Abweichungen von der Soll-Konzentration oder beim Setzen einer neuen Soll-Konzentration kann das Regelventil automatisch nachgeregelt werden.

In einer Ausführung steuert die Steuereinheit das Ventil zur Reduktion des Gasdruckes und/oder das Regelventil zur variablen Einstellung des Gasflusses in Abhängigkeit des Atemzyklus. Bei dieser Ausführung fließen nicht nur die Durchflussmessungen als Steuergrößen ein, sondern das Öffnen und Drosseln des/der Ventile erfolgt auch in Abhängigkeit des Atemzyklus. Die Steuerung erfolgt so, dass nur dann Sauerstoff abgegeben wird, wenn er auch tatsächlich gebraucht wird, wodurch Sauerstoff gespart werden kann.

Das erfindungsgemäße Verfahren zum Betrieb eines Beatmungsgeräts und/oder Anästhesiegeräts, bei dem ein Atemgas durch Mischen eines unter Druck stehenden medizinischen Gases, z.B. Sauerstoff, mit durch ein Gebläse geförderter Luft bereitgestellt wird, umfassend eine Gasleitung für das medizinische Gas, und eine Luftleitung, die in einer gemeinsamen Atemgasleitung münden, ist dadurch gekennzeichnet, dass die Einmündung des medizinischen Gases in die gemeinsame Atemgasleitung stromabwärts des Gebläses erfolgt und dass die Zufuhr des medizinischen Gases in die Atemgasleitung mit einem Regelventil, vorzugsweise einem Proportionalventil, in der Gasleitung gesteuert wird und dass das Atemgas mit einem Regelventil in der Atemgasleitung dosiert wird.

In einer Ausführungsform wird die Förderung des medizinischen Gases in die Atemgasleitung ausschließlich durch den, gegebenenfalls reduzierten, Eigendruck des in die Gasleitung eintretenden Gases bewirkt.

In einer Ausführungsform erfolgt die Steuerung des Regelventils in der Gasleitung in Abhängigkeit der Messwerte eines in der Gasleitung vorgesehenen Durchflusssensors und eines in der Atemgasleitung vorgesehenen Durchflusssensors.

Der Hochdrucksauerstoff wird vorzugsweise über ein Reduktionsventil in der Gasleitung einer Flussmessung zugeführt und von dort über ein erstes ansteuerbares Proportionalventil in eine Atemgasleitung geführt. Der Blower liefert Luft vorzugsweise über ein Rückschlagventil, ebenso in diese Atemgasleitung. Ausgangsseitig der Atemgasleitung befindet sich eine zweite Flussmessung und ein zweites Proportionalventil (das Beatmungsventil).

Beide Proportionalventile werden in Realtime und vom Atemrhytmus des Patienten angesteuert. Die Steuerung misst und vergleicht beide Flussmessungen, so dass entsprechend der voreingestellten Werte die richtige Relation Sauerstoff zum Gesamtfluss zugemischt wird.

In einer Ausführungsform werden die Messwerte der Durchflusssensoren in Echtzeit ausgewertet und zueinander in Beziehung gestellt und wird in Abhängigkeit dieser Messwertverarbeitung das Regelventil in Echtzeit angesteuert wird. So können geringfügige Abweichungen von Soll-Werten sofort festgestellt und über eine entsprechende Ansteuerung des Regelventils ausgeglichen werden.

In einer Ausführungsform erfolgt die Steuerung des Regelventils in der Gasleitung in Abhängigkeit des Atemzyklus.

In einer Ausführungsform wird in der Gasleitung der eingangsseitig anliegende Druck des medizinischen Gases durch ein Ventil reduziert, das in Bezug auf das Regelventil stromaufwärts angeordnet ist.

In einer Ausführungsform wird das Ventil zur Reduktion des Gasdruckes in Abhängigkeit des Atemzyklus derart gesteuert wird, dass das Ventil gedrosselt wird, wenn ausgangsseitig kein Atemzug an der Atemgasleitung anliegt.

In einer Ausführungsform wird der Atemzyklus über einen ausgangsseitig in der Atemgasleitung angeordneten Gasdrucksensor bestimmt. Es aber selbstverständlich auch denkbar, dass der Atemzyklus auf andere Art bestimmt wird, z.B. mittels elastischen Brustbänder zur Messung der Ausdehnung des Brustkastens, Messung des Sauerstoffgehalts zwischen Atem- und Ausatemluft, etc..

In einer Ausführungsform wird ein Rückstrom des medizinischen Gases in die Luftleitung durch ein zwischen dem Gebläse und der Einmündung der Luftleitung in die Atemgasleitung angeordnetes Rückschlagventil verhindert.

Das Beatmungs- und/oder Anästhesiegerät kann selbstverständlich auch eine Ausgabeeinheit, bzw. ein Display, einen Rechner, ein Steuerprogramm, sowie weitere internen Sensoren und natürlich wenigstens einen Beatmungsanschluss für Patienten aufweisen.

Weitere Ausbildungen der Erfindung sind in den Figuren und in den abhängigen Patentansprüchen angegeben. Die Bezugszeichenliste ist Bestandteil der Offenbarung. Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

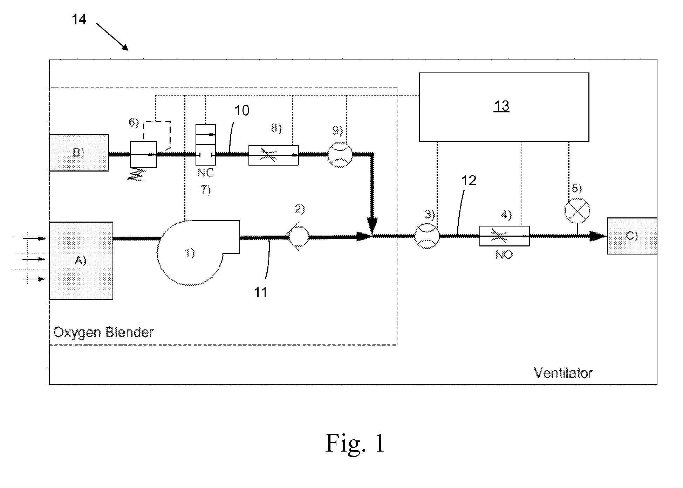

Es zeigen dabei
Fig.1 - das Blockdiagramm eines erfindungsgemäßen Beatmungsgeräts.

Figur 1 zeigt ein erfindungsgemäßes Beatmungsgerät 14, z.B. ein (mobiles) Heimbeatmungsgerät oder ein auf Intensivstationen oder in Operationssälen zum Einsatz kommendes Beatmungs- oder Anästhesiegerät. Das Beatmungsgerät 14 ist funktionell in zwei Bereiche gegliedert, eine Mischvorrichtung, in der ein medizinisches Gas, wie z.B. Sauerstoff, Stickstoffmonoxid, diverse andere Anästhetika, gasförmige Schmerzmittel, etc., mit Luft vermischt wird, und einen Ventilationsteil, über den das entstehende Gasgemisch dem Patienten als Atemgas mit entsprechendem Druck und Durchfluss (Mengendosierung) zugeführt wird. Das Beatmungsgerät 14 umfasst somit eine Gasleitung 10 und eine Luftleitung 11, die in einer gemeinsamen Atemgasleitung 12 münden.

Ein Gaseinlass B, zum Beispiel in Form einer Anschlussstelle für eine Hochdruckgasflasche oder ein Anschluss für ein Hochdruckgasnetz, mündet in eine Gasleitung 10, in der das medizinische Gas gefördert wird. Eingangsseitig besitzt die Gasleitung 10 ein Ventil 6, üblicherweise ein Reduktionsventil, mit dem der Gasdruck stromabwärts reduziert wird. Vorzugsweise ist dieses Ventil variabel einstellbar, so dass auch der strom abwärtige Gasdruck beliebig regelbar ist. Ein Regelventil 8, ein Proportionalventil, bestimmt den Durchfluss des medizinischen Gases in Richtung der gemeinsamen Atemgasleitung 12. Ein Durchflussmesser 9 ist in der Gasleitung 10 integriert, der im dargestellten Ausführungsbeispiel stromabwärts des Regelventils 8 angeordnet ist. Für Zwecke der Sicherheit ist zwischen dem Reduktionsventil 6 und dem Regelventil 8 ein Sicherheitsventil 7 vorgesehen, das beim Ausfall oder bei einer falschen Ansteuerung des Ventils 6 zum Einsatz kommt, d.h. bei Überschreiten eines Druck-Schwellwertes in der Gasleitung 10 schließt. In einer bevorzugten Ausgestalltung wird auch das Sicherheitsventil 7 von der Steuereinheit 13 angesteuert. Das Reduktionsventil 6 kann ebenfalls von der Steuereinheit 13 angesteuert werden oder als ein pneumatischer Druckregler ausgebildet sein.

Die Förderung des medizinischen Gases in der Gasleitung 10 erfolgt ausschließlich durch den Eigendruck des in einem externen Druckreservoir gespeicherten und über den Gaseinlass B zur Verfügung gestellten Gases. Es sind somit keine aktiven Fördereinrichtungen in der Gasleitung 10 nötig.

Ein Lufteinlass A, der vorzugsweise mit einem Filter zur Reinigung der angesaugten Luft ausgestattet ist, mündet in einer Luftleitung 11, in der ein Gebläse 1 für die aktive Förderung der angesaugten Luft sorgt. Stromabwärts des Gebläses 1 sitzt ein Rückschlagventil 2 in der Luftleitung 11 und verhindert den Rückstrom des medizinischen Gases bzw. des Gasgemisches in Richtung Lufteinlass A. Die beiden Leitungen 10 und 11 gehen in eine gemeinsame Atemgasleitung 12 über, die bereits das Gasgemisch gewünschter Zusammensetzung enthält. Die Atemgasleitung 12 umfasst einen Durchflusssensor 3 und ein Regelventil 4, vorzugsweise ein Proportionalventil, und einen Drucksensor 5. Das Regelventil 4 dient der Mengendosierung des Gasgemisches entsprechend den patientenspezifischen, medizinischen Anforderungen.

Alle regelbaren Ventile und Sensoren in den Leitungen sowie das Gebläse 1 sind steuer- bzw. signaltechnisch mit einer Steuereinheit 13 verbunden. Die Steuer- bzw. Signalleitungen sind in Fig. 1 strichliert dargestellt. Ein wesentliches Merkmal einer Ausführungsform der Erfindung besteht darin, dass das Regelventil 8 in der Gasleitung 10 von der Steuereinheit 13 derart gesteuert wird, dass das in der Atemgasleitung 12 geförderte Gasgemisch die gewünschte Zusammensetzung aufweist. Zu diesem Zweck liest die Steuereinheit 13 die ständig, bevorzugt kontinuierlich, aufgenommenen Messwerte der Durchflusssensoren 3 und 9 aus, wobei aus diesen Messwerten eine Regelgröße bestimmt wird, die zur Regelung des Regelventils 8 herangezogen wird. Nach Aufbereitung der Messwerte mithilfe von Kalibrierdaten und/oder Berücksichtigung weiterer Parameter, wie zum Beispiel Temperatur, Druck, Feuchtigkeit, etc., spielt insbesondere das Verhältnis der Durchflussmenge in der Gasleitung 10 zur Durchflussmenge in der Gasleitung 12 eine wesentliche Rolle. Dieses Verhältnis bestimmt den Anteil des medizinischen Gases im Gasgemisch. (Bei Sauerstoff muss selbstverständlich berücksichtigt werden, dass Luft bereits einen natürlichen Anteil an Sauerstoff enthält.) Weichen die Ergebnisse der Messauswertung von einem Soll-Wert bzw. einer Soll-Zusammensetzung des Atemgases ab, kann das Regelventil 8 leicht nachgeregelt werden, wodurch eine Feedbackschleife entsteht.

In einer besonders bevorzugten Ausführungsform werden die Messwerte der Durchflusssensoren kontinuierlich in Echtzeit ausgewertet und als Regelgröße bereitgestellt. Ebenso werden die Ventile gemäß dieser sich ständig ändernden Regelgröße in Echtzeit angesteuert, so dass die Ventile auch dem Atemzyklus angepasst werden können. Wenn zum Beispiel kein Atemzug am Ausgang C der Atemgasleitung anliegt, kann das Regelventil 8 gedrosselt werden, um nicht unnötig Sauerstoff zuzuführen. Ebenso kann das Regelventil 4 in der Atemgasleitung in diesen Phasen gedrosselt werden. Um bei einer Drosselung des Regelventils 8 die Entstehung eines übermäßigen Druckes vor dem Regelventil 8 in der Gasleitung 10 zu vermeiden, ist vorzugsweise auch das Ventil 6 zur Reduzierung des Gasdruckes regelbar ausgebildet. Entsprechend der Drosselung des Regelventils 8 wird nun auch das Ventil 6 in entsprechender Weise gedrosselt, damit der Druck zwischen den beiden Ventilen 6, 8 nicht ansteigt. Andernfalls würde bei einer nachfolgenden Öffnung des Regelventils 8 - verursacht durch den Überdruck -, übermäßig viel Sauerstoff in den Luftstrom zugeführt werden, was zu einer Verfälschung der gewünschten Gaszusammensetzung in der Atemgasleitung führen würde.

In einer bevorzugten Variante wird auch das Gebläse 1 in Abhängigkeit des Atemzyklus gesteuert.

Zur Erfassung des Atemzyklus wird ein Drucksensor 5 ausgangsseitig in der Atemgasleitung 12 herangezogen. Die Ventile 6, 8 und/oder 4 werden somit in Abhängigkeit der Messwerte des Drucksensors 5 in Echtzeit angesteuert. Selbstverständlich kann der Atemzyklus mit jeder beliebigen Methode erfasst werden. Am Atemgasauslass C des Beatmungsgerätes 14 wird üblicherweise eine Beatmungsmaske über eine flexible Leitung angeschlossen.

### Bezugszeichenliste

1 - Gebläse
2 - Rückschlagventil
3 - Durchflusssensor
4 - Regelventil
5 - Drucksensor
6 - Ventil
7 - Sicherheitsventil
8 - Regelventil
9 - Durchflusssensor
10 - Gasleitung
11 - Luftleitung
12 - Atemgasleitung
13- Steuereinheit
14 - Beatmungsgerät
A - Lufteinlass
B - Gaseinlass
C - Atemgasauslass

## Patentansprüche

1. Beatmungsgerät (14) und/oder Anästhesiegerät mit einer Mischvorrichtung zum Mischen eines unter Druck stehenden medizinischen Gases, z.B. Sauerstoff, mit durch ein Gebläse (1) angesaugter Luft, umfassend eine Gasleitung (10) für das medizinische Gas, und eine Luftleitung (11), die in einer gemeinsamen Atemgasleitung (12) münden, **dadurch gekennzeichnet, dass** sich die Einmündung der Gasleitung (10) in die gemeinsame Atemgasleitung (12) stromabwärts des Gebläses (1) befindet und dass die Gasleitung (10) ein Regelventil (8), vorzugsweise ein Proportionalventil, zur variablen Einstellung des Gasflusses aufweist und dass in der gemeinsamen Atemgasleitung (12) ein Regelventil (4), vorzugsweise ein Proportionalventil, zur Dosierung des Atemgases vorgesehen ist.

2. Beatmungsgerät und/oder Anästhesiegerät nach Anspruch 1, **dadurch gekennzeichnet, dass** in der Gasleitung (10) ein Durchflusssensor (9) zur Messung des Gasflusses vorgesehen ist und in der Atemgasleitung (12) ein Durchflusssensor (3) zur Messung des Atemgasflusses vorgesehen ist.

3. Beatmungsgerät und/oder Anästhesiegerät nach einem der vorhergehenden Ansprüche, **dadurch gekennzeichnet, dass** in der Gasleitung (10) eingangsseitig ein Ventil (6) zur Reduktion des Gasdruckes vorgesehen ist, wobei das Regelventil (8) stromabwärts davon angeordnet ist, **wobei** das Ventil (6) zur Reduktion des Gasdruckes vorzugsweise variabel einstellbar ist.

4. Beatmungsgerät und/oder Anästhesiegerät nach einem der vorhergehenden Ansprüche, **dadurch gekennzeichnet, dass** in der Gasleitung (11), vorzugsweise zwischen dem Ventil (6) zur Reduktion des Gasdruckes und dem Regelventil (8) zur variablen Einstellung des Gasflusses, ein Sicherheitsventil (7) vorgesehen ist.

5. Beatmungsgerät und/oder Anästhesiegerät nach einem der vorhergehenden Ansprüche, **dadurch gekennzeichnet, dass** das Gebläse (1) in der Luftleitung angeordnet ist.

6. Beatmungsgerät und/oder Anästhesiegerät nach einem der vorhergehenden Ansprüche, **dadurch gekennzeichnet, dass** in der Luftleitung (11) zwischen dem Gebläse (1) und der Einmündung der Luftleitung (11) in die Atemgasleitung (12) ein Rückschlagventil (2) vorgesehen ist, das den Rückstrom von Gas in Richtung Gebläse (1) verhindert.

7. Beatmungsgerät und/oder Anästhesiegerät nach einem der vorhergehenden Ansprüche, **dadurch gekennzeichnet, dass** in der Atemgasleitung (12) ausgangsseitig ein Gasdrucksensor (5) vorgesehen ist.

8. Beatmungsgerät und/oder Anästhesiegerät nach einem der vorhergehenden Ansprüche, **dadurch gekennzeichnet, dass** die Förderung des medizinischen Gases in die Atemgasleitung (12) ausschließlich durch den, gegebenenfalls reduzierten, Eigendruck des in die Gasleitung (10) eintretenden Gases bewirkt wird.

9. Beatmungsgerät und/oder Anästhesiegerät nach einem der vorhergehenden Ansprüche, **dadurch gekennzeichnet, dass** das Regelventil (8) und die Durchflussmesser (3, 9) des Beatmungsgeräts und/oder Anästhesiegeräts (14) mit einer Steuereinheit (13) verbunden sind, die das Regelventil (8) in Abhängigkeit der Messwerte des Durchflusssensors (9) in der Gasleitung (10) und des Durchflusssensors (3) in der Atemgasleitung (12) steuert.

10. Beatmungsgerät und/oder Anästhesiegerät nach einem der vorhergehenden Ansprüche, **dadurch gekennzeichnet, dass** Steuereinheit (13) das Ventil (6) zur Reduktion des Gasdruckes und/oder das Regelventil (8) zur variablen Einstellung des Gasflusses in Abhängigkeit des Atemzyklus steuert.

11. Verfahren zum Betrieb eines Beatmungsgeräts und/oder Anästhesiegeräts, bei dem ein Atemgas durch Mischen eines unter Druck stehenden medizinischen Gases, z.B. Sauerstoff, mit durch ein Gebläse (1) geförderter Luft bereitgestellt wird, umfassend eine Gasleitung (10) für das medizinische Gas, und eine Luftleitung (11), die in einer gemeinsamen Atemgasleitung (12) münden, **dadurch gekennzeichnet, dass** die Einmündung des medizinischen Gases in die gemeinsame Atemgasleitung (12) stromabwärts des Gebläses (1) erfolgt und die Zufuhr des medizinischen Gases in die Atemgasleitung (12) mit einem Regelventil (8), vorzugsweise einem Proportionalventil, in der Gasleitung (10) gesteuert wird und dass das Atemgas mit einem Regelventil (4), vorzugsweise einem Proportionalventil, in der Atemgasleitung (12) dosiert wird.

12. Verfahren nach Anspruch 11, **dadurch gekennzeichnet, dass** die Förderung des medizinischen Gases in die Atemgasleitung (12) ausschließlich durch den, gegebenenfalls reduzierten, Eigendruck des in die Gasleitung (10) eintretenden Gases bewirkt wird, **und/oder dass** die Steuerung des Regelventils (8) in der Gasleitung (10) in Abhängigkeit der Messwerte eines in der Gasleitung (10) vorgesehenen Durchflusssensors (9) und eines in der Atemgasleitung (12) vorgesehenen Durchflusssensors (3) erfolgt.

13. Verfahren nach einem der vorhergehenden Ansprüche, **dadurch gekennzeichnet, dass** die Messwerte der Durchflusssensoren (3, 9) in Echtzeit ausgewertet und zueinander in Beziehung gestellt werden und dass in Abhängigkeit dieser Messwertverarbeitung das Regelventil (8) in Echtzeit angesteuert wird, **und/oder dass vorzugsweise** die Steuerung des Regelventils (8) in der Gasleitung (10) in Abhängigkeit vom Atemzyklus erfolgt **und/oder dass** weiter vorzugsweise der Atemzyklus über einen ausgangsseitig in der Atemgasleitung (12) angeordneten Gasdrucksensor (5) bestimmt wird.

14. Verfahren nach einem der vorhergehenden Ansprüche, **dadurch gekennzeichnet, dass** in der Gasleitung (10) der eingangsseitig anliegende Druck des medizinischen Gases durch ein Ventil (6) reduziert wird, das in Bezug auf das Regelventil (8) stromaufwärts angeordnet ist.

15. Verfahren nach einem der vorhergehenden Ansprüche, **dadurch gekennzeichnet, dass** das Ventil (6) zur Reduktion des Gasdruckes in Abhängigkeit des Atemzyklus derart gesteuert wird, dass das Ventil (6) gedrosselt wird, wenn ausgangsseitig kein Atemzug an der Atemgasleitung (12) anliegt.

16. Verfahren nach einem der vorhergehenden Ansprüche, **dadurch gekennzeichnet, dass** ein Rückstrom des medizinischen Gases in die Luftleitung (11) durch ein zwischen dem Gebläse (1) und der Einmündung der Luftleitung (11) in die Atemgasleitung (12) angeordnetes Rückschlagventil (2) verhindert wird.
